Europäisches Patentamt

European Patent Office

Office européen des brevets

(19)

(11) **EP 0 690 105 B1**

(12) **EUROPÄISCHE PATENTSCHRIFT**

(45) Veröffentlichungstag und Bekanntmachung des Hinweises auf die Patenterteilung:
09.09.1998 Patentblatt 1998/37

(51) Int Cl.6: **C09C 1/00**, C09D 7/12, C09D 11/00, C08K 9/02, C03C 4/02, C04B 33/14, A61K 7/00

(21) Anmeldenummer: 95109309.5

(22) Anmeldetag: 16.06.1995

(54) **Glanzpigmente mit stickstoffhaltigen Metallschichten**

Brilliant pigments exhibiting nitrogen containing metallic layers

Pigments brillants comportant des couches métalliques contenant de l'azote

(84) Benannte Vertragsstaaten:
CH DE FR GB IT LI

(30) Priorität: 23.06.1994 DE 4421933

(43) Veröffentlichungstag der Anmeldung:
03.01.1996 Patentblatt 1996/01

(73) Patentinhaber: BASF Aktiengesellschaft
67063 Ludwigshafen (DE)

(72) Erfinder:
• Schmid, Raimund, Dr.
D-67435 Neustadt (DE)
• Mronga, Norbert, Dr.
D-69221 Dossenheim (DE)
• Ochmann, Harald, Dr.
D-67125 Dannstadt-Schauernheim (DE)
• Adel, Jörg, Dr.
D-67071 Ludwigshafen (DE)

(56) Entgegenhaltungen:
EP-A- 0 332 071       EP-A- 0 338 428
EP-A- 0 353 544       WO-A-93/12182
FR-A- 2 687 162       US-A- 5 246 493

• DATABASE WPI Week 9007 Derwent Publications Ltd., London, GB; AN 90-049655 & JP-A-02 004 955 (FUJIYOSHI) , 9.Januar 1990

**Beschreibung**

Die vorliegende Erfindung betrifft neue Glanzpigmente auf der Basis von beschichteten plättchenförmigen Substraten mit stickstoffhaltigen Metallschichten, welche dadurch gekennzeichnet sind, daß sie stickstoffhaltige Metallschichten mit Eisen, Cobalt, Nickel, Chrom, Molybdän, Wolfram, Vanadium, Mangan, Rhenium, Ruthenium, Osmium, Rhodium und/oder Iridium sowie gewünschtenfalls zusätzlich eine oder mehrere im wesentlichen aus farblosem oder selektiv absorbierendem Metalloxid bestehende Schichten enthalten.

Weiterhin betrifft die Erfindung die Herstellung dieser Pigmente und Pigmentmischungen sowie ihre Verwendung zum Einfärben von Lacken, Druckfarben, Tinten, Kunststoffen, Gläsern und keramischen Produkten.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Insbesondere an einen metallischen Eindruck vermittelnden, silbernen Glanzpigmenten, die vor allem für die Einfärbung von Lakken und Druckfarben eingesetzt werden, besteht ein großer Bedarf, der durch die bereits bekannten Pigmente nicht zufriedenstellend gedeckt werden kann.

In den DE-A-41 41 069 und 42 17 511 sowie den älteren deutschen Patentanmeldungen DE-A-43 13 541 und DE-A-44 05 492 werden mit Metall- und gewünschtenfalls Metalloxidschichten belegte metallische und silikatische Pigmente beschrieben, die zwar einen metallischen Effekt bewirken, jedoch im Gegensatz zu dem typischen von Metallen wie Aluminium vermittelten "kalten" bläulichen Silberton einen "wärmeren" rötlichen Silberton zeigen. Weiterhin erfordern diese Pigmente in der Regel eine äußere Schutzschicht zur Erhöhung ihrer Korrosionsbeständigkeit.

Schließlich sind Pigmente bekannt, die Titannitridschichten oder titannitridhaltige Oxidschichten aufweisen. So werden in der US-A-5 246 493 titannitridbeschichtete Titanplättchen mit gold-gelber Farbe und in der JP-A-4 955/1990 titannitridbeschichtete Metallblättchen beschrieben. In den EP-A-338 428 und 332 071 werden Aluminium- bzw. Glimmerpigmente mit Titandioxidbeschichtung, in die durch reduzierende Behandlung mit Ammoniak Titannitride eingebaut werden, offenbart.

Der Erfindung lag die Aufgabe zugrunde, neue Glanzpigmente mit Metalliceffekt bereitzustellen, welche die genannten Nachteile nicht zeigen und vorteilhafte Anwendungseigenschaften aufweisen.

Demgemäß wurden die eingangs definierten Glanzpigmente gefunden.

Als besonders bevorzugte Variante wurden Glanzpigmente gefunden, die

A)    eine innere, im wesentlichen aus farblosem oder selektiv absorbierendem Metalloxid bestehende Schicht,

B)    eine stickstoffhaltige Metallschicht mit Eisen, Cobalt, Nickel, Chrom, Molybdän, Wolfram, Vanadium, Mangan, Rhenium, Ruthenium, Osmium, Rhodium und/oder Iridium als metallischem Bestandteil und

C)    gewünschtenfalls eine äußere, im wesentlichen aus farblosem oder selektiv absorbierendem Metalloxid bestehende Schicht

aufweisen.

Weiterhin wurde ein Verfahren zur Herstellung der Glanzpigmente gefunden, welches dadurch gekennzeichnet ist, daß man

die stickstoffhaltigen Metallschichten durch Gasphasenzersetzung von Carbonylen der Metalle Eisen, Cobalt, Nikkel, Chrom, Molybdän, Wolfram, Vanadium, Mangan, Rhenium, Ruthenium, Osmium, Rhodium und/oder Iridium in Gegenwart von Ammoniak und

die Metalloxidschichten, falls gewünscht, in üblicher Weise durch Gasphasenzersetzung flüchtiger Metallverbindungen in Gegenwart von Sauerstoff und/oder Wasserdampf oder naßchemisch durch Hydrolyse geeigneter Metallverbindungen

auf die gegebenenfalls bereits mit einer inneren Metalloxidschicht belegten Substratteilchen aufbringt.

Schließlich wurde die Verwendung der Glanzpigmente zur Einfärbung von Lacken, Druckfarben, Tinten, Kunststoffen, Gläsern, keramischen Produkten und Zubereitungen der dekorativen Kosmetik gefunden.

Für die erfindungsgemäßen Glanzpigmente können als Substrate sowohl silikatische als auch metallische Plättchen sowie auch Mischungen solcher Plättchen verwendet werden.

Als silikatische Substrate sind dabei insbesondere helle bzw. weiße Glimmer, vor allem Schuppen von naß vermahlenem Muskovit geeignet. Selbstverständlich können auch andere natürliche Glimmer wie Phlogopit und Biotit, künstliche Glimmer, Talk- und Glasschuppen eingesetzt werden.

Als metallische Substrate sind alle für Metalleffektpigmente bekannten Metalle und Legierungen in Plättchenform geeignet. Z.B. kommen neben Stahl, Kupfer und seinen Legierungen wie Messing und Bronzen vor allem Aluminium und seine Legierungen wie Aluminiumbronze in Betracht.

Bevorzugt sind Aluminiumflakes, die in einfacher Weise durch Herausstanzen aus Aluminiumfolie oder nach gängigen Verdüsungs- und Mahltechniken herzustellen sind.

So sind beispielsweise Aluminiumpigmente geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschrieben, durch oxidative Behandlung entfernt werden.

Die zum Einsatz kommenden Substratteilchen können bereits mit einer ersten Schicht (A) aus farblosem oder farbigem Metalloxid belegt sein. Das ist insbesondere dann vorteilhaft, wenn nicht schwarze, sondern bunte Interferenzfarben zeigende Pigmente erhalten werden sollen. Geeignet sind die üblicherweise zur Beschichtung von Glanzpigmenten verwendeten Oxide wie Silicium-, Zinn-, Zink-, Aluminium- und Chromoxid, besonders Eisen- und Zirkondioxid und ganz besonders Titandioxid sowie auch Mischungen dieser Oxide.

Metalloxidbeschichtete silikatische und metallische Pigmente sind allgemein bekannt und im Fall beschichteter Glimmerpigmente auch unter den Namen Iriodin® (E. Merck, Darmstadt), Flonac® (Kemira Oy, Pori, Finnland) und Mearlin® (Mearl Corporation, New York) im Handel. Die Herstellung der metalloxidbeschichteten Glimmerpigmente kann bekanntermaßen aus wäßriger Phase (DE-A-14 67 468, DE-A-25 22 572) oder aus der Gasphase (EP-A-45 851, DE-A-32 37 264) erfolgen, die entsprechenden Metallpigmente können ebenfalls durch Beschichtung aus der Gasphase (EP-A-33 457) oder aus alkoholischer Lösung (DE-A-35 34 477, EP-A-328 906) oder aus wäßrig-organischer Phase (DE-A-44 05 492) hergestellt werden.

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von im allgemeinen etwa 0,1 bis 5 µm, insbesondere etwa 0,5 µm. Ihre spezifische freie Oberfläche (BET) beträgt im allgemeinen 0,1 bis 5 $m^2$/g.

Sind die Substratteilchen mit einer ersten Metalloxidschicht (A) belegt, so liegt die Dicke dieser Schicht üblicherweise in dem für die klassischen Glanzpigmente bekannten Bereich, d.h. bei Pigmenten auf silikatischer Basis in der Regel bei 20 bis 400 nm, bevorzugt bei 35 bis 250 nm, und bei Pigmenten auf metallischer Basis im allgemeinen bei 20 bis 800 nm, vorzugsweise bei 70 bis 600 nm.

Wesentlich für die erfindungsgemäßen Glanzpigmente sind die stickstoffhaltigen Metallschichten (B), die direkt auf die unbeschichteten oder vorteilhaft auf die bereits mit Metalloxid beschichteten Substratteilchen aufgebracht sind.

Die erfindungsgemäßen Schichten (B) enthalten als wesentlichen Bestandteil eines oder mehrere Metalle, die flüchtige Carbonyle bilden, nämlich Eisen, Cobalt, Nickel, Chrom, Molybdän, Wolfram, Vanadium, Mangan, Rhenium, Ruthenium, Osmium, Rhodium und/oder Iridium, wobei Wolfram, Cobalt und Nickel bevorzugt sind, Chrom und Molybdän besonders bevorzugt sind und Eisen ganz besonders bevorzugt ist.

Außerdem enthalten die Metallschichten (B) in der Regel etwa 0,01 bis etwa 22, vorzugsweise etwa 0,1 bis etwa 15 Gew.-% Stickstoff.

Im allgemeinen wird der enthaltene Stickstoff, zumindest teilweise, in Form von Metallnitriden vorliegen.

Die stickstoffhaltigen Metallschichten (B) können vorteilhaft nach dem erfindungsgemäßen Verfahren durch Gasphasenzersetzung der Metallcarbonyle in Gegenwart von Ammoniak aufgebracht werden (chemical vapor deposition, CVD).

Die so erhältlichen, dunklen, nichtselektiv absorbierenden Schichten (B) zeichnen sich durch gleichmäßigen, homogenen, filmartigen Aufbau aus. Je nach Schichtdicke sind sie mehr oder weniger lichtdurchlässig und tragen auch zur Interferenz bei.

Im allgemeinen werden die Schichten (B) 1 bis 100 nm, bevorzugt 0,2 bis 20 nm dick sein, wobei insbesondere bei Schichtdicken von ≤ 10 nm transparente bis semitransparente Schichten vorliegen, welche die Interferenzfarbe eines mit Metalloxid beschichteten Substrats unter Ausbildung besonders brillanter Farbtöne verstärken können.

Beschichtet man ein mit einer nicht interferenzfähigen Schicht, beispielsweise einer dünnen Titandioxidschicht (< 40 nm), belegtes Glimmerpigment mit einer erfindungsgemäßen stickstoffhaltigen Eisenschicht, so erhält man ein Pigment mit silberner Körperfarbe, das im Unterschied zu den nach den bekannten Herstellungsverfahren zu erhal-

tenden Eisen- und TiO$_2$-beschichteten Glimmerpigmenten bei der Applikation in Lack einen "kälteren", bläulichen Metalliceffekt bewirkt.

Neben den vorteilhaften koloristischen Effekten zeichnen sich die stickstoffhaltigen Metallschichten (B) im Vergleich zu den bekannten gleichartigen Pigmenten zusätzlich durch eine deutliche Verbesserung der chemischen Beständigkeit z.B. gegen Sauerstoff (Luft), Wasser und Wasserdampf aus.

Die erfindungsgemäßen Glanzpigmente können noch eine Deckschicht (C) aufweisen, die aus farblosen oder selektiv absorbierenden Metalloxiden aufgebaut wird. Besonders geeignet sind hierfür die für die inneren Metalloxidschichten (A) beispielhaft genannten Metalloxide. In Abhängigkeit von der Herstellung dieser Schichten (z.B. naßchemisches Aufbringen und Trocknen) können die Metalloxidschichten noch geringe Mengen Wasser enthalten, die Metalloxide also teilweise als Oxidhydrate vorliegen.

Die Dicke der Schicht (C) ist an sich nicht kritisch, im allgemeinen beträgt sie etwa 1 bis 400 nm, insbesondere 5 bis 200 nm.

Die Schicht (C) kann einen zusätzlichen Schutz des Pigments bewirken und auch zur Interferenz des Pigments beitragen und dabei die Interferenzfarbenreihe an der durch das mit (A) und (B) beschichtete Substrat bestimmten Stelle fortsetzen. Dies ist beispielsweise der Fall, wenn Zirkon- oder Titanoxid als Schicht (C) aufgebracht werden. Besteht dagegen die Schicht (C) im wesentlichen aus Siliciumoxid, so wird sich diese Schicht im Anwendungsmedium (z.B. Lacken, Druckfarben oder Tinten), das einen ähnlichen Brechungsindex aufweist, koloristisch kaum bemerkbar machen.

Farbige Metalloxide wie Eisen- und Chromoxid werden die Interferenzfarbe des Mehrschichtsystems durch Beimischen ihrer Absorptionsfarbe modifizieren und mit zunehmender Schichtdicke schließlich überdecken.

Bei dem erfindungsgemäßen Verfahren zur Herstellung der neuen Glanzpigmente werden die einzelnen Beschichtungen vorteilhaft aus der Gasphase durch Zersetzung geeigneter Ausgangsverbindungen der Metalle auf die Substratteilchen, die bereits eine erste Metalloxidschicht aufweisen können, aufgebracht. Die Metalloxidschichten können auch naßchemisch, z.B. wie in der älteren deutschen Patentanmeldung DE-A-44 05 492 beschrieben, abgeschieden werden.

Die Gasphasenbeschichtung wird zweckmäßigerweise in einem beheizbaren Wirbelschichtreaktor, wie er beispielsweise in der EP-A-45 851 beschrieben ist, durchgeführt, in dem die Substratteilchen zunächst mit einem Wirbelgas fluidisiert und auf die für die Zersetzung der jeweiligen Metallverbindung erforderliche Temperatur von in der Regel 100 bis 600°C, bevorzugt 150 bis 400°C, erhitzt werden. Die in einem vorgeschalteten Verdampfergefäß unter Verwendung eines geeigneten Trägergases verdampften Metallverbindungen sowie die gegebenenfalls zur Zersetzung benötigten Gase werden dann über getrennte Düsen eingetragen.

Die neuen stickstoffhaltigen Metallschichten (B) werden erfindungsgemäß durch Zersetzung der Metallcarbonyle in Gegenwart von Ammoniak aufgebracht.

Besonders geeignet sind hierfür z.B. Wolframhexacarbonyl, Dicobaltoctacarbonyl, Nickeltetracarbonyl, vor allem Chrom- und Molybdänhexacarbonyl und insbesondere Eisenpentacarbonyl.

Wie für CVD-Verfahren allgemein üblich, ist es auch für die Herstellung der Schichten (B) zweckmäßig, wenn die verdampfte Metallverbindung ≤ 5 Vol.-%, bevorzugt ≤ 2 Vol.-%, der Gesamtgasmenge im Reaktor ausmacht.

Der als Reaktionsgas benötigte Ammoniak kann dem inerten Wirbelgas (insbesondere Stickstoff oder Argon) in Mengen von 0,01 bis 99 Vol.-% beigemischt werden. Der Ammoniak könnte im Extremfall sogar selbst als Wirbelgas dienen; dies ist jedoch aus Gründen der Abluftentsorgung nicht empfehlenswert. Ein Maximalgehalt des Wirbelgases von in der Regel 15 bis 20 Vol.-% Ammoniak hat sich als sinnvoll erwiesen, besonders bevorzugt ist ein Ammoniakgehalt von 0,01 bis 5 Vol.-%.

In Abhängigkeit von der Ammoniakkonzentration und der gewählten Zersetzungstemperatur sowie auch vom eingesetzten Metallcarbonyl wird mehr oder weniger Stickstoff in die Schicht (B) eingebaut. Generell ist festzustellen, daß der Stickstoffgehalt bei einem gegebenen Metallcarbonyl mit steigender Temperatur und steigender Ammoniakkonzentration zunimmt.

Zweckmäßigerweise wird dem Wirbelgas nach beendeter Beschichtung und Abkühlung auf Raumtemperatur etwas Luft zugemischt, um eventuell vorhandene pyrophore Anteile der Metallschicht (B) zu passivieren, so daß die Schicht (B) in der Regel auch geringe Mengen Sauerstoff enthält.

Die gewünschten Metalloxidschichten (C) und auch (A) können vorteilhaft, wie z.B. aus der EP-A-571 836 bekannt, durch oxidative Zersetzung von Metallcarbonylen oder durch hydrolytische Zersetzung von Metallalkoholaten auf den Substratteilchen abgeschieden werden. Beispiele für geeignete flüchtige Metallverbindungen sind Titan- und Zirkontetra-n- und isopropanolat, Chromhexacarbonyl und Eisenpentacarbonyl.

Weiterhin können zum Aufbau der Metalloxidschichten, wie in der älteren deutschen Patentanmeldung DE-A-44 03 678 beschrieben, auch Metallorganyle oxidativ zersetzt werden. Im einzelnen seien z.B. Zinntetraalkyle, -alkenyle und -aryle sowie gemischte Zinnarylalkyle und -alkylalkenyle, wie Zinndiallyldibutyl, Zinntetraamyl, Zinntetra-n-propyl, Bis(tri-n-butylzinn)oxid und vor allem Zinntetra-n-butyl und Zinntetramethyl, und Zinkdialkyle, wie Zinkdiethyl, genannt.

Die Metalloxidschichten können auch naßchemisch durch Hydrolyse geeigneter Metallverbindungen aufgebracht werden. Dies empfiehlt sich insbesondere für im wesentlichen aus Silicium- und/oder Aluminiumoxid bzw. -oxidhydrat bestehende Schichten, die besonders vorteilhaft nach dem in der älteren deutschen Patentanmeldung DE-A-44 05 492 beschriebenen Verfahren durch saure oder bevorzugt basische Hydrolyse organischer Silicium- und/oder Aluminiumverbindungen, bei denen die organischen Reste über Sauerstoffatome an die Metalle gebunden sind (also z.B. Acetylacetonate oder Alkoholate, insbesondere $C_1$-$C_4$-Alkanolate), in Gegenwart eines organischen Lösungsmittels, in dem die Metallverbindungen löslich sind und die mit Wasser mischbar sind (also z.B. Alkohole, Ketone, Ether, Carbonsäureamide), hergestellt werden können. Bei einer besonders bevorzugten Ausführungsform dieses Verfahrens werden Tetraethoxysilan und/oder Aluminiumtriisopropanolat in Gegenwart von Isopropanol und einer wäßrigen Ammoniaklösung als Katalysator (und selbstverständlich der Substratteilchen) hydrolysiert, wobei schrittweise auf Rückflußtemperatur erhitzt wird.

Mit Hilfe des erfindungsgemäßen Verfahrens können die neuen Glanzpigmente in einfacher Weise in großen Mengen hergestellt werden. Es werden vollständig umhüllte Pigmentteilchen mit hoher Qualität der einzelnen Beschichtungen und gutem Deckvermögen sowie guter Korrosionsbeständigkeit erhalten.

Gewünschtenfalls können die erfindungsgemäßen Glanzpigmente, die auf metallischen Substratteilchen basieren, zur Desagglomerierung und Glättung einem zusätzlichen Veredelungsschritt durch schonendes Aufmahlen in einer Kugelmühle oder vergleichbaren Apparaten unterzogen werden.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und besonders von Tinten und Druckfarben, u.a. Sicherheitsdruckfarben, und vor allem von Lacken, insbesondere Automobillacken, wo aufgrund des ausgezeichneten Deckvermögens der Pigmente auf die sonst bei Glimmerpigmenten notwendige Grundierung verzichtet werden kann. Insbesondere die Pigmente mit silbernen Farbtönen können hervorragend als Ersatz von reinen (bzw. passivierten) Aluminiumpigmenten in lösungsmittelhaltigen und vor allem wäßrigen Metalliclacken eingesetzt werden. Bei der Applikation im Druck sind alle industrieüblichen Druckverfahren, z.B. Siebdruck, Tiefdruck, Bronzierdruck, Flexodruck und Offsetdruck, geeignet.

Beispiele

Herstellung, Anwendung und Beurteilung von erfindungsgemäßen Glanzpigmenten

Die Herstellung der erfindungsgemäßen Glanzpigmente erfolgte jeweils in einem von außen beheizbaren Wirbelschichtreaktor aus Glas mit einem Durchmesser von 8 cm und einer Höhe von 80 cm mit Glasfrittenboden und oben eingehängten, mit einem Stickstoff-Jet abreinigenden Filterstrümpfen und zwei seitlich oberhalb des Frittenbodens eingebrachten Düsen zur Gaseinleitung.

Zur Beurteilung der Koloristik der erhaltenen Pigmente wurden je 0,4 g der Pigmentproben in 3,6 g eines Polyester-Mischlackes mit 21 Gew.-% Feststoffanteil eingerührt und 2 Minuten lang im Red Devil dispergiert. Mit einer Rakel (160 μm Naßfilmdicke) wurden anschließend auf einem schwarzweißen Karton Abzüge der pigmentierten Lacke angefertigt. Die Messung der CIELAB-Werte erfolgte nach dem Trocknen des Films mit einem Gonio-Spektralphotometer Multiflash M 45 der Firma Optronik (Berlin) bei einer Winkel-Differenz von 20°, 45° bzw. 115° zum Glanzwinkel. Die Angaben der Farbwerte (L, a*, b*) beziehen sich auf die Normlichtart D 65 und einen Betrachtungswinkel von 25°. Dabei entspricht L der Helligkeit, a* dem Rot- bzw. Grünanteil und b* dem Blau- bzw. Gelbanteil. H ist der Farbwinkel [°] und C* das Chroma. Gemessen wurde über schwarzem Untergrund an einfach abgerakelten Proben.

Zur Anwendung der Pigmente im Bronzierdruck wurden Papierbögen zunächst im Offset-Verfahren mit einem nichtpigmentierten Klebefirnis (Bronzierfirnis) aus 95 Gew.-% Leinölfirnis und phenolmodifiziertem Kolophoniumharzester und 5 Gew.-% Polyvinyltoluol bedruckt und dann sofort in die Bronzierstation weitergeführt, wo sie mit dem Pigment bestäubt wurden. Überschüssiges Pigmentpulver wurde anschließend mit einer Samtrakel entfernt.

Zur Anwendung der Pigmente im Siebdruck wurden 10 g Pigment in 90 g einer handelsüblichen Bindemittellösung (22,5 g PVC-Mischpolymerisat Laroflex® MP45, 4,5 g Methoxypropylacetat, 13,5 g n-Hexyldiglykol, 49,5 g Butylglykol) eingerührt. Die so hergestellte Siebdruckfarbe wurde mit einer handelsüblichen Siebdruckmaschine (Siebmaschenweite 112 bis 150 μm) auf gestrichenes, titandioxidbeschichtetes Papier in einer Schichtdicke von 45 μm aufgebracht und an der Luft getrocknet.

Beispiele 1 bis 7

600 g des jeweils in Tabelle 1 angegebenen, mit Titandioxid beschichteten Glimmerpigments wurden unter Verwirbelung mit Insgesamt a l/h Stickstoff auf T°C erhitzt. 400 l/h des Wirbelgases wurden dabei über eine temperierte (20°C im Fall von $Fe(CO)_5$, 80°C im Fall von $Mo(CO)_6$) Vorlage mit x g Metallcarbonyl geleitet, das so in t h in den Reaktor überführt wurde und dort bei gleichzeitiger Einleitung von b l/h Ammoniak unter Bildung von sich filmartig auf den Substratteilchen abscheidendem, stickstoffhaltigem Metall und Kohlenmonoxid zersetzt wurde. Nach beendeter Metallabscheidung und Abkühlen auf Raumtemperatur wurde den Wirbelgasen zur Passivierung eventuell vorhandener pyrophorer Anteile der Metallschicht etwas Luft zugesetzt.

Einzelheiten zu diesen Versuchen sowie deren Ergebnisse sind in Tabelle 1 aufgeführt.

Die koloristischen Daten der Pigmente sind in Tabelle 2 (Meßwinkel a: 20°, b: 45°, c: 115°) zusammengestellt.

Zum Vergleich sind dort auch die Daten für das analog Beispiel 1, jedoch in Abwesenheit von Ammoniak mit Eisen beschichtete und den Stand der Technik repräsentierende Glimmerpigment V1 angegeben.

Als Standard wurde eine mit einem feinteiligen Aluminiumpulver (mittlere Teilchengröße, BET-Oberfläche 3,5 m²/g; Alu VP 501, Fa. Eckart, Fürth) erhaltene Lackierung vermessen.

Zur Beurteilung des "metallischen Charakters" der mit den erfindungsgemäßen Pigmenten und dem Vergleichspigment V1 erhaltenen Lackierungen wurden als Maß für die koloristische Abweichung vom Standard die $\Delta$E-Werte nach

$$\Delta E = \sqrt{\Delta H^2 + \Delta C^{*2} + \Delta L^2}$$

berechnet. Bei nahezu identischer Koloristik sind $\Delta$E-Werte von 0 bis 5 zu erwarten.

Die Ergebnisse zeigen, daß die erfindungsgemäßen Pigmente zwar noch vom Standard abweichen, jedoch deutlich "metallischer" sind als die gemäß dem Stand der Technik zu erhaltenden Pigmente (die $\Delta$E-Werte sind mindestens um 10 Einheiten kleiner).

Tabelle 1

| Bsp. | Substrat | a l/h $N_2$ | T [°C] | x g Me(CO)$_x$ | t [h] | b l/h $NH_3$ | Metallgehalt [Gew.-%] | N-Gehalt [Gew.-%] bez. auf Pigment / Metallschicht | | Anwendung [c] |
|---|---|---|---|---|---|---|---|---|---|---|
| 1 | X[a] | 1800 | 200 | 218 Fe(CO)$_5$ | 10 | 5 | Fe: 9,6 | 0,36 | 3,6 | L, B, S: "kalter" metallischer Glanz |
| 2 | X | 1600 | 220 | 450 Fe(CO)$_5$ | 24 | 10 | Fe: 15,5 | 1,03 | 6,2 | L: wie Bsp. 1 |
| 3 | X | 1400 | 240 | 330 Fe(CO)$_5$ | 14 | 10 | Fe: 9,5 | 0,47 | 4,7 | L: wie Bsp. 1 |
| 4 | X | 1600 | 250 | 290 Fe(CO)$_5$ | 8 | 15 | Fe: 10,0 | 0,36 | 3,6 | L: wie Bsp. 1 |
| 5 | X | 1800 | 250 | 70 Fe(CO)$_5$ | 8 | 5 | Fe: 3,8 | 0,15 | 3,8 | L: wie Bsp. 1 |
| 6 | X | 1400 | 225 | 206 Mo(CO)$_6$ | 35 | 10 | Mo: 8,5 | 0,65 | 7,1 | L: "kalter" metallischer Glanz, etwas dunkler als entspr. eisenbeschichtete Pigmente |
| 7 | Y[b] | 1800 | 200 | 218 Fe(CO)$_5$ | 10 | 5 | Fe: 9,0 | 0,4 | 4,2 | L: farbstark violett |
| V1 | X | 1800 | 200 | 218 Fe(CO)$_5$ | 10 | − | Fe: 9,5 | − | − | L, B, S: rötlicher Silberton |

[a] X ≙ Iriodin® 9103 Rutil Sterling Silber WR (Merck) – silbern

[b] Y ≙ Mearlin® Hi Lite Violet 539 X (Mearl) – violette Interferenzfarbe

[c] L ≙ Lack; B ≙ Bronzierdruck, S ≙ Siebdruck

Tabelle 2a: Meßwinkel 20°

| Bsp. | H [°] | C* | L | a* | b* | ΔH | ΔC* | ΔL | Δa* | Δb* | ΔE |
|------|-------|------|--------|-------|-------|-------|------|--------|-------|-------|-------|
| Al | 253,66 | 0,54 | 123,87 | -0,15 | -0,52 | | | | | | |
| 1 | 241,91 | 3,12 | 96,40 | -1,47 | -2,76 | -0,27 | 2,59 | -27,47 | -1,32 | -2,24 | 27,59 |
| 2 | 234,93 | 2,61 | 97,55 | -1,50 | -2,13 | -0,39 | 2,07 | -26,32 | -1,35 | -1,62 | 26,40 |
| 3 | 192,75 | 1,02 | 96,61 | -1,00 | -0,23 | -0,75 | 0,49 | -27,26 | -0,85 | 0,29 | 27,27 |
| 4 | 215,13 | 1,22 | 97,62 | -0,99 | -0,70 | -0,53 | 0,68 | -26,25 | -0,84 | -0,18 | 26,26 |
| 5 | 165,37 | 1,15 | 102,09 | -1,11 | 0,29 | -1,10 | 0,61 | -21,77 | -0,96 | 0,81 | 21,81 |
| V1 | 122,87 | 1,98 | 84,01 | -1,08 | 1,66 | -1,88 | 1,44 | -39,86 | -0,92 | 2,18 | 39,93 |

Tabelle 2b: Meßwinkel 45°

| Bsp. | H [°] | C* | L | a* | b* | ΔH | ΔC* | ΔL | Δa* | Δb* | ΔE |
|------|-------|------|-------|-------|-------|-------|-------|--------|-------|-------|-------|
| Al | 261,39 | 1,74 | 64,54 | -0,26 | -1,72 | | | | | | |
| 1 | 252,11 | 1,84 | 42,89 | -0,57 | -1,75 | -0,29 | 0,1 | -21,65 | -0,30 | -0,03 | 21,65 |
| 2 | 248,68 | 1,59 | 45,48 | -0,58 | -1,48 | -0,37 | -0,15 | -19,06 | -0,32 | 0,24 | 19,07 |
| 3 | 106,59 | 0,64 | 44,21 | -0,18 | 0,61 | -2,06 | -1,10 | -20,33 | 0,08 | 2,33 | 20,47 |
| 4 | 4,18 | 0,17 | 44,74 | 0,17 | 0,01 | 0,84 | -1,58 | -19,80 | 0,43 | 1,73 | 19,88 |
| 5 | 106,59 | 0,64 | 44,21 | -0,18 | 0,61 | -2,06 | -1,10 | -20,33 | 0,08 | 2,33 | 20,47 |
| V1 | 114,65 | 0,51 | 30,73 | -0,21 | 0,46 | -1,81 | -1,23 | -33,82 | 0,05 | 2,19 | 33,89 |

Tabelle 2c: Meßwinkel 115°

| Bsp. | H [°] | C* | L | a* | b* | ΔH | ΔC* | ΔL | Δa* | Δb* | ΔE |
|---|---|---|---|---|---|---|---|---|---|---|---|
| Al | 252,36 | 1,47 | 33,64 | -0,45 | -1,41 | | | | | | |
| 1 | 273,27 | 1,60 | 23,56 | 0,09 | -1,60 | 0,56 | 0,13 | -10,08 | 0,54 | -0,19 | 10,09 |
| 2 | 261,32 | 2,45 | 23,77 | -0,37 | -2,42 | 0,30 | 0,98 | - 9,87 | 0,08 | -1,02 | 9,93 |
| 3 | 268,55 | 1,96 | 21,57 | -0,05 | -1,95 | 0,48 | 0,48 | -12,07 | 0,40 | -0,55 | 12,09 |
| 4 | 331,31 | 1,09 | 24,04 | 0,96 | -0,52 | 1,61 | -0,38 | - 9,60 | 1,40 | 0,88 | 9,74 |
| 5 | 292,15 | 0,86 | 24,16 | 0,32 | -0,80 | 0,77 | -0,62 | - 9,48 | 0,77 | 0,61 | 9,53 |
| Vl | 264,46 | 2,35 | 12,17 | -0,23 | -2,34 | 0,39 | 0,87 | -21,47 | 0,22 | -0,93 | 21,49 |

**Patentansprüche**

1. Glanzpigmente auf der Basis von beschichteten plättchenförmigen Substraten, mit stickstoffhaltigen Metallschichten, dadurch gekennzeichnet, daß sie stickstoffhaltige Metallschichten mit Eisen, Cobalt, Nickel, Chrom, Molybdän, Wolfram, Vanadium, Mangan, Rhenium, Ruthenium, Osmium, Rhodium und/oder Iridium als metallischem Bestandteil sowie gewünschtenfalls zusätzlich eine oder mehrere im wesentlichen aus farblosem oder selektiv absorbierendem Metalloxid bestehende Schichten enthalten.

2. Glanzpigmente nach Anspruch 1, bei denen der enthaltene Stickstoff im wesentlichen in Form von Metallnitriden vorliegt.

3. Glanzpigmente nach Anspruch 1 oder 2, die

   A) eine innere, im wesentlichen aus farblosem oder selektiv absorbierendem Metalloxid bestehende Schicht,

   B) eine stickstoffhaltige Metallschicht mit Eisen, Cobalt, Nickel, Chrom, Molybdän, Wolfram, Vanadium, Mangan, Rhenium, Ruthenium, Osmium, Rhodium und/oder Iridium als metallischem Bestandteil und

   C) gewünschtenfalls eine äußere, im wesentlichen aus farblosem oder selektiv absorbierendem Metalloxid bestehende Schicht

   aufweisen.

4. Glanzpigmente nach den Ansprüchen 1 bis 3, bei denen die Metalloxidschichten im wesentlichen aus Titan-, Zirkon-, Zinn-, Zink-, Silicium-, Aluminium-, Chrom- und/oder Eisenoxid bestehen.

5. Glanzpigmente nach den Ansprüchen 1 bis 4, bei denen das Substrat aus silikatischen oder metallischen Plättchen oder deren Mischungen besteht.

6. Verfahren zur Herstellung von Glanzpigmenten gemäß den Ansprüchen 1 bis 5, dadurch gekennzeichnet, daß man

   die stickstoffhaltigen Metallschichten durch Gasphasenzersetzung von Carbonylen der Metalle Eisen, Cobalt, Nickel, Chrom, Molybdän, Wolfram, Vanadium, Mangan, Rhenium, Ruthenium, Osmium, Rhodium und/oder Iridium in Gegenwart von Ammoniak und

   die Metalloxidschichten in üblicher Weise durch Gasphasenzersetzung flüchtiger Metallverbindungen in Gegenwart von Sauerstoff und/oder Wasserdampf oder naßchemisch durch Hydrolyse geeigneter Metallverbindungen

   auf die gegebenenfalls bereits mit einer inneren Metalloxidschicht belegten Substratteilchen aufbringt.

7. Verwendung von Glanzpigmenten gemäß den Ansprüchen 1 bis 5 zur Einfärbung von Lacken, Druckfarben, Tinten, Kunststoffen, Gläsern, keramischen Produkten und Zubereitungen der dekorativen Kosmetik.

**Claims**

1. Luster pigments based on coated plateletlike substrates, with nitrogen-containing metal layers, characterized in that they comprise nitrogen-containing metal layers having iron, cobalt, nickel, chromium, molybdenum, tungsten, vanadium, manganese, rhenium, ruthenium, osmium, rhodium and/or iridium as the metal and if desired additionally one or more layers consisting essentially of colorless or selectively absorbing metal oxide.

2. Luster pigments as claimed in claim 1 wherein the nitrogen is essentially present in the form of metal nitrides.

3. Luster pigments as claimed in claim 1 or 2 comprising

   A) an inner layer consisting essentially of colorless or selectively absorbing metal oxide,

B) a nitrogen-containing metal layer having iron, cobalt, nickel, chromium, molybdenum, tungsten, vanadium, manganese, rhenium, ruthenium, osmium, rhodium and/or iridium as the metal, and

C) if desired an outer layer consisting essentially of colorless or selectively absorbing metal oxide.

4. Luster pigments as claimed in any of claims 1 to 3 wherein the metal oxide layers consist essentially of titanium oxide, zirconium oxide, tin oxide, zinc oxide, silicon oxide, aluminum oxide, chromium oxide and/or iron oxide.

5. Luster pigments as claimed in any of claims 1 to 4 wherein the substrate consists of silicatic or metallic platelets or mixtures thereof.

6. A process for producing luster pigments as claimed in any of claims 1 to 5, characterized in that it comprises applying to the substrate particles, or to an inner metal oxide layer already existing thereon, the nitrogen-containing metal layers by gas phase decomposition of carbonyls of the metals iron, cobalt, nickel, chromium, molybdenum, tungsten, vanadium, manganese, rhenium, ruthenium, osmium, rhodium and/or iridium in the presence of ammonia and the metal oxide layers in a conventional manner by gas phase decomposition of volatile metal compounds in the presence of oxygen and/or water vapor or wet-chemically by hydrolysis of suitable metal compounds.

7. The use of luster pigments as claimed in any of claims 1 to 5 for coloring paints, inks, including printing inks, plastics, glasses, ceramic products and decorative cosmetic preparations.

**Revendications**

1. Pigments brillants à base de substrats sous forme de plaquettes enduites comportant des couches métalliques contenant de l'azote, caractérisés en ce qu'ils contiennent des couches métalliques contenant de l'azote avec, en tant que constituant métallique, du fer, du cobalt, du nickel, du chrome, du molybdène, du tungstène, du vanadium, du manganèse, du rhénium, du ruthénium, de l'osmium, du rhodium et/ou de l'iridium, ainsi éventuellement qu'une ou plusieurs couches supplémentaires constituées pour l'essentiel d'oxyde métallique incolore ou absorbant de façon sélective.

2. Pigments brillants selon la revendication 1 pour lesquels l'azote contenu se présente essentiellement sous forme de nitrures métalliques.

3. Pigments selon la revendication 1 ou 2 présentant

A) une couche interne constituée essentiellement d'oxyde métallique incolore ou absorbant de façon sélective,
B) une couche métallique contenant de l'azote avec, en tant que constituant métallique, du fer, du cobalt, du nickel, du chrome, du molybdène, du tungstène, du vanadium, du manganèse, du rhénium, du ruthénium, de l'osmium, du rhodium et/ou de l'iridium, et
C) une éventuelle couche externe constituée essentiellement d'oxyde métallique incolore ou absorbant de façon sélective.

4. Pigments brillants selon l'une quelconque des revendications 1 à 3 pour lesquels les couches d'oxyde métallique sont constituées pour l'essentiel d'oxyde de titane, de zirconium, d'étain, de zinc, de silicium, d'aluminium, de chrome et/ou de fer.

5. Pigments brillants selon l'une quelconque des revendications 1 à 4 pour lesquels le substrat est constitué de plaquettes métalliques ou silicatées ou bien de leurs mélanges.

6. Procédé de préparation de pigments brillants selon l'une quelconque des revendications 1 à 5, caractérisé en ce que l'on dépose,

sur les particules de substrat éventuellement déjà recouvertes d'une couche interne d'oxyde métallique, les couches métalliques contenant de l'azote par décomposition en phase gazeuse de composés carbonylés des métaux fer, cobalt, nickel, chrome, molybdène, tungstène, vanadium, manganèse, rhénium, ruthénium, osmium, rhodium, et/ou iridium, en présence d'ammoniaque et les couches d'oxyde métallique de façon usuelle par décomposition en phase gazeuse de composés métalliques volatils, en présence d'oxygène et/ou de vapeur d'eau, ou par voie chimique humide au moyen d'une hydrolyse de composés métalliques appropriés.

7. Utilisation de pigments brillants selon l'une quelconque des revendications 1 à 5 pour la coloration de laques, d'encres d'impression, d'encres, de matières plastiques, de verres, de produits céramiques et de préparations pour la cosmétique décorative.